# FASCICULE DE BREVET EUROPEEN

(11) **EP 3 439 706 B1**
(45) Date de publication et mention de la délivrance du brevet: **19.02.2025**
(21) Numéro de dépôt: 17711782.7
(22) Date de dépôt: 15.03.2017
(51) Int. Cl.: A61L 27/20, A61L 27/52, A61L 27/26, A61L 27/46, A61L 27/48, A61L 27/54, A61K 51/00, A61L 24/00, A61L 24/04, A61L 24/08

(54) **PRODUIT PERMETTANT LA FORMATION D'UN IMPLANT THERAPEUTIQUE DANS LE CORPS D'UN SUJET - IMPLANT ET COMPOSITION ASSOCIES**
PRODUKT ZUR BILDUNG EINES THERAPEUTISCHEN IMPLANTATS IM KÖRPER EINER PERSON, ZUGEHÖRIGES IMPLANTAT UND ZUSAMMENSETZUNG
PRODUCT ENABLING THE FORMATION OF A THERAPEUTIC IMPLANT INSIDE THE BODY OF A SUBJECT - ASSOCIATED IMPLANT AND COMPOSITION

(30) Priorité: 07.04.2016 FR 1653049
(43) Date de publication de la demande: 13.02.2019
(73) Titulaire: Gelnesis, 14610 Cairon (FR)
(72) Inventeur: LE BAIL, Michaël, 14200 Hérouville Saint Clair (FR)
(74) Mandataire: Ipside
(86) Numéro de dépôt international: PCT/IB2017/051482
(87) Numéro de publication internationale: WO 2017/175081

(56) Documents cités:
- JP-A- H0 920 651
- JP-A- H04 230 636
- PHAECHAMUD THAWATCHAI ET AL: "Solvent exchange-inducedin situforming gel comprising ethyl cellulose-antimicrobial drugs", INTERNATIONAL JOURNAL OF PHARMACEUTICS, ELSEVIER, NL, vol. 494, no. 1, 21 August 2015 (2015-08-21), pages 381 - 392, XP029276816, ISSN: 0378-5173, DOI: 10.1016/J.IJPHARM.2015.08.047
- ROBERT MORHARD ET AL: "Development of enhanced ethanol ablation as an alternative to surgery in treatment of superficial solid tumors", SCIENTIFIC REPORTS, vol. 7, no. 1, 18 August 2017 (2017-08-18), XP055501130, DOI: 10.1038/s41598-017-09371-2
- ANNE DOMPMARTIN ET AL: "Radio-opaque ethylcellulose-ethanol is a safe and efficient sclerosing agent for venous malformations", EUROPEAN RADIOLOGY, SPRINGER, BERLIN, DE, vol. 21, no. 12, 7 August 2011 (2011-08-07), pages 2647 - 2656, XP019978128, ISSN: 1432-1084, DOI: 10.1007/S00330-011-2213-4
- MARTIN SCHUMACHER ET AL: "Treatment of venous malformations: First experience with a new sclerosing agent A multicenter study", EUROPEAN JOURNAL OF RADIOLOGY, ELSEVIER SCIENCE, NL, vol. 80, no. 3, 28 December 2010 (2010-12-28), pages e366 - e372, XP028102778, ISSN: 0720-048X, [retrieved on 20110120], DOI: 10.1016/J.EJRAD.2010.12.074

## Description

La présente invention concerne un produit permettant par injection la formation d'un implant solide dans le corps d'un sujet, une composition obtenue à partir du produit, l'implant formé par injection du produit ainsi qu'un procédé de fabrication du produit,

L'article intitulé mise au point d'un gel sclérosant alcoolique d'éthylcellulose utilisé pour le traitement des malformations veineuses, publié en mars 2001 dans la revue Pharm Clin, vol 20, n°1 en mars 2001 décrit une solution contenant de l'eau, de l'éthanol et de l'éthylcellulose. À température ambiante, cette solution est suffisamment fluide pour être injectée dans une veine, au niveau d'une malformation veineuse à traiter. Au contact du sang, la solution forme un précipité solide qui obture le vaisseau ou la veine et permet le passage de l'alcool du précipité vers la paroi veineuse qui est ainsi traitée par l'action sclérosante de l'éthanol, par ailleurs connue.

La publication intitulée «radio-opaque ethylcellulose-ethanol is a safe and efficient sclerosing agent for venous malformations», publiée le 7 août 2011, dans la revue European Radiology, SPRINGER, vol. 21, n° 12, pages 2647 à 2656 décrit une émulsion d'un gel d'éthylcellulose et de Lipiodol^{®}, un agent de contraste iodé qui est injectée et utilisée en tant qu'agent sclérosant. L'agent de contraste permet de localiser la masse formée dans l'organisme par l'émulsion qui a réagi avec le sang. L'agent de contraste reste piégé dans l'implant ainsi formé ; il permet simplement la localisation de ce dernier.

Par ailleurs, la publication « Treatment of venous malformations : first experience with a new sclerosing agent -- A multicenter study », publiée le 28 décembre 2010, dans la revue European Journal of Radiology, ELSEVIER SCIENCE, vol. 80, n°3, pages e366-e372, décrit une étude multicentrique concernant un gel d'éthanol et d'éthylcellulose, utilisé en tant qu'agent sclérosant. Ce gel est injecté et forme une masse qui du fait de l'éthanol qu'elle contient présente un pouvoir sclérosant. L'injection du gel est précédée d'une injection d'un produit de contraste afin de détecter la position the l'aiguille et d'évaluer la quantité de gel à injecter. Le déplacement de l'agent de contraste autour de la masse formée par précipitation du gel au contact du sang, permet de localiser cette dernière. La quantité d'éthanol qui passe de la masse à l'organisme s'est révélée être faible.

Hormis l'effet sclérosant conféré par l'éthanol, la masse formée par précipitation du gel d'éthylcellulose et d'éthanol au contact du sang, ne présente aucun autre effet thérapeutique.

Un but de la présente invention est donc de proposer un produit, comme défini dans les revendications, qui permet d'obtenir une composition injectable apte d'une part, à former un corps solide ou implant une fois injectée dans l'organisme d'un sujet et d'autre part à délivrer un composé actif ou substance pharmacologiquement active.

Un autre but de la présente invention est de proposer un produit, comme défini dans les revendications, qui permet d'obtenir une composition injectable apte à former un corps solide ou implant une fois injectée dans l'organisme d'un sujet et qui présente une durée de conservation fiable.

Un autre but de la présente invention est de proposer un produit tel que précité qui puisse être localisé dans le corps du sujet, pendant l'injection et/ou post injection.

Un autre but de la présente invention est de proposer un produit injectable comme défini dans les revendications, qui forme un implant dans l'organisme du sujet dans lequel il est injecté et qui présente au moins une action thérapeutique avec une quantité réduite de substance active.

Un autre but de l'invention est de proposer un produit comme défini dans les revendications qui forme un implant dans l'organisme du sujet dans lequel il est injecté et qui libère au moins une substance active sur une zone limitée dans l'organisme du sujet.

Un autre but de la présente invention est de proposer un produit injectable comme défini dans les revendications qui forme un implant dans l'organisme du sujet dans lequel il est injecté et qui puisse prendre une forme adaptée à l'organisme du sujet, en particulier lorsque l'implant est formé dans une cavité, par exemple osseuse, et qu'il peut également servir également de matériau de comblement.

Un autre but de la présente invention est de proposer un produit injectable comme défini dans les revendications, apte à former dans l'organisme du sujet un implant dont l'empreinte auto-formée, épouse l'anfractuosité ou la cavité à combler dans laquelle le produit a été injecté.

Un autre but de la présente invention est de proposer un produit injectable **,** comme défini dans les revendications, qui est susceptible de former un implant dans l'organisme du sujet dans lequel il est injecté et qui présente plusieurs activités pharmacologiques et/ou thérapeutiques.

Un autre but de la présente invention est de proposer un produit injectable **,** comme défini dans les revendications, qui est susceptible de former un implant dans l'organisme du sujet dans lequel il est injecté, ledit implant ne nécessitant pas un acte chirurgical pour être retiré.

La présente invention propose ainsi un comme défini dans les revendications permettant par injection la formation d'un implant solide dans le corps d'un sujet, ledit produit comprenant une composition de base, pharmaceuticalement acceptable, qui contient au moins un solvant et au moins un premier polysaccharide soluble dans ledit solvant, ladite composition de base est apte à former un précipité au contact d'une solution aqueuse éventuellement saline.

De manière caractéristique selon l'invention, il comprend, outre, une quantité donnée d'au moins une substance active, pharmaceuticalement acceptable sous forme de poudre, choisie parmi :
- les agents bactéricides/antiseptiques/antifongiques ioniques et solubles ou miscibles dans ledit solvant tels que l'éosine et le bleu de méthylène et l'iodure de potassium ;
- les antibiotiques, les facteurs de croissance, l'hydroxyapatite, les hormones, les agents anti tumoraux, les agents antimitotiques, les inhibiteurs de la topo isomérase, les agents anti-cancéreux et les mélanges d'au moins deux desdites substances actives
en ce que ladite quantité de substance active est mélangée à ladite composition de base ou conditionnée séparément de ladite composition de base, en ce que le mélange formé par ladite composition de base et ladite quantité de substance active présente une viscosité qui permet l'injection dudit mélange et en ce que ledit mélange forme un précipité au contact d'une solution aqueuse éventuellement saline permettant de former ledit implant dans l'organisme du sujet dans lequel il est injecté, et de diffuser ladite substance active dans un milieu environnant l'implant: et en ce que ledit premier polysaccharide est l'éthylcellulose et ledit solvant est choisi parmi l'éthanol et les mélanges eau-éthanol comportant au moins 50% en volume d'éthanol et notamment au moins 90% en volume d'éthanol.

C'est le mérite de la Demanderesse que d'avoir constaté qu'il est possible d'ajouter à la composition de base une substance pharmaceuticalement active apte à se dissoudre dans cette dernière et distincte du solvant de la composition de base. Le mélange de la composition de base et de la substance active présente une viscosité qui le rend injectable à température ambiante ou après avoir éventuellement été chauffé. Une fois le mélange formé par les composants du produit injecté dans l'organisme d'un sujet, le mélange précipite au contact de tout tissu hydraté et de toute solution aqueuse (sang et/ou de la lymphe) et forme une masse que l'on appelle implant et qui contient la substance active. Cet implant se compose d'un précipité floconneux, il est par conséquent souple et peut donc former une masse qui épouse les contours de la cavité dans laquelle le mélange formé par le produit a été injecté. Le produit forme un implant dont l'empreinte auto-formée, épouse l'anfractuosité ou la cavité à combler dans laquelle le produit a été injecté. L'implant étant souple, il n'abîme pas les tissus l'environnant.

L'action pharmaceutique de l'implant peut venir de la substance active uniquement ; elle peut également être au moins partiellement conférée par le solvant. Si le solvant et la substance active ont une même activité thérapeutique, il est possible de doser judicieusement la quantité de substance active et la quantité de solvant. Le solvant va rester dans l'implant tandis que la substance active va diffuser dans le milieu environnant l'implant et produire un effet thérapeutique sur une plus grande zone. On obtient ainsi une même activité thérapeutique étalée dans le temps et sur une zone plus vaste. Si le solvant et la substance active ont une activité pharmaceutique différente, l'implant présentera plusieurs activités pharmaceutiques étalées dans le temps et/ou sur des zones différentes de l'organisme du sujet.

Le ou les agents bactéricides/antiseptiques/antifongiques ioniques sont sous forme de poudre et de préférence solubles dans ledit solvant. L'ajout d'un tel solide ne modifie pas la viscosité de la composition de base.

Selon un mode de réalisation particulièrement avantageux, le produit comprend toujours au moins une substance active choisie parmi les agents bactéricides/antiseptiques/antifongiques et notamment le bleu de méthylène, l'éosine et l'iodure de potassium, ce dernier pouvant être introduit sous forme de teinture d'iode.

Ces composés ont également le mérite d'être colorés. Lorsqu'ils passent dans l'organisme, ils colorent les cellules dans lesquelles ils vont pénétrer. Ces cellules peuvent se nécroser et nécessiter une résection ultérieure. Cette dernière est facile à mettre en œuvre du fait de la coloration des tissus qui doivent être enlevés.

De plus, les composés précités sont également asséchant et vont compléter le rôle du solvant quand celui-ci est l'éthanol. L'éthanol lui ne peut migrer dans l'organisme du fait de la grande stabilité du précipité qu'il forme avec l'éthylcellulose, notamment.

Lorsque la composition de base contient de l'éthanol éventuellement mélangé à 10% ou moins d'eau et de l'éthylcellulose et que la substance active est ionique et soluble l'éthanol et/ou l'eau, on obtient deux effets thérapeutiques sur deux zones. En effet, le mélange éthylcellulose-éthanol forme du fait des liaisons hydrogène de l'éthanol et de l'éthylcellulose un mélange stable et homogène. La substance active n'est liée à ce mélange que par les forces de Van der Waals qui sont moindres ; elle diffuse donc dans le milieu ambiant.

Cet effet est notamment obtenu lorsque l'on utilise en tant que substance active du bleu de méthylène et/ou de l'éosine et/ou de l'iodure de potassium pouvant se trouver sous forme de teinture d'iode, et en particulier du bleu de méthylène ou de l'éosine. Ces substances ont des propriétés asséchantes et antiseptiques et font également fonction de colorant. L'ensemble implant + tissu cible nécrosé est donc visible à l'œil nu ou lors d'un examen échographique ou endoscopique et peut être retiré facilement, par un acte chirurgical, par exemple. La substance active diffuse hors de l'implant, éliminant les germes potentiels sur une plus grande zone, stimulant de plus le système immunitaire du sujet. Elle diffuse également dans les cellules touchant l'implant par osmose. Le solvant reste lui dans l'implant mais lorsqu'il est hydrophile il attire l'eau contenue dans le cytoplasme des cellules avoisinant l'implant par osmose, notamment. Le premier polysaccharide et le deuxième polysaccharide, lorsqu'il est présent, amplifient la migration d'eau du cytoplasme des cellules avoisinantes vers l'implant: les cellules vidées de leur eau cytoplasmique meurent. Le bleu de méthylène, l'éosine et l'iodure de potassium ou la teinture d'iode renforcent encore le phénomène de déshydratation des cellules au contact de l'implant. Il y a donc une synergie entre le ou les polysaccharide(s) et le bleu de méthylène, l'éosine, l'iodure de potassium ou la teinture d'iode. Lorsque le solvant est hydrophile, comme c'est le cas de l'éthanol pur ou en solution dans l'eau, la synergie existe entre ce solvant et au moins un ou les deux éléments précités (à savoir le ou les polysaccarides et les colorants précités)

L'implant va donc déshydrater les cellules environnantes (vider le cytoplasme des cellules) et provoquer ainsi une sclérose du tissu ciblé implanté.

Il en est de même pour toute substance active ionique ou toute combinaison de substances actives ioniques. Toute substance active ou tout mélange de substances actives est de préférence, utilisé sous forme d'une poudre qui est mélangée à la composition de base pour former une solution ou une suspension ou conservée séparément de cette dernière de manière à éviter sa dégradation éventuelle du fait de réaction avec les autres constituants de la composition de base.

Lorsque le produit comprend de l'hydroxyapatite, celle-ci peut former une suspension avec la composition de base. La Demanderesse a en effet mis en évidence que l'hydroxyapatite forme une solution stable dans une composition de base contenant de l'éthanol éventuellement mélangé avec de l'eau tel que décrit ultérieurement, et de l'éthylcellulose.

Enfin, certains composants du produit de l'invention peuvent conférer plusieurs fonctions à l'implant formé. Ainsi, les agents bactéricides/antiseptiques/antifongiques ioniques précités sont également des colorants ; ils sont assimilés à des agents de contraste car ils permettent de localiser l'implant lors d'une échographie ou d'une célioscopie.

De même, certains anticancéreux peuvent servir également d'agents de contraste lorsqu'ils demeurent dans l'implant. Il en est de même des radionucléides qui peuvent à la fois avoir une action thérapeutique/pharmacologique et servir d'agents de contraste.

La quantité de substance active n'est pas limitée selon l'invention ; à titre d'exemple, elle peut représenter plus de 50% en masse de la composition de base. On obtient ainsi un implant qui présente une concentration en substance active homogène dans tout volume.

L'implant peut être formé dans toute cavité ou dans tout organe ou tissu, mou ou dur, accessible par une aiguille ou un cathéter. Ainsi, le mélange issu des constituants du produit de l'invention peut être injecté, par exemple, dans une veine ou un vaisseau sanguin, dans une tumeur éventuellement cancéreuse, un kyste ou dans une cavité osseuse de manière à la combler et/ou à favoriser sa reconstruction par formation de matière osseuse nouvelle. La taille de l'implant est facile à maîtriser car elle dépend directement de la quantité de composition selon l'invention injectée dans l'organisme du sujet.

Dans le cas d'une tumeur notamment cancéreuse, il est possible en injectant le mélange obtenu par le produit de l'invention à la périphérie de la tumeur de scléroser les vaisseaux sanguins alimentant cette dernière. On obtient ainsi un premier effet thérapeutique du fait de la substance active qui peut être un anticancéreux et un second effet thérapeutique par asséchement (destruction) des vaisseaux et des cellules tumorales par contact, par exemple, lorsque le solvant est de l'éthanol ou un mélange eau-éthanol et que le produit contient également du bleu de méthylène ou de l'éosine ou de l'iodure de potassium en une quantité suffisante pour obtenir un effet desséchant, sclérosant et destructeur. De plus, on obtient un implant coloré qui colore également le tissu dans lequel il a été implanté (tumeur implantée, par exemple). L'ensemble est visible à l'œil ce qui permet de localiser à la fois l'implant et la tumeur, lorsque ce dernier s'est formé dans une tumeur.

Ledit premier polysaccharide est l'éthylcellulose. On obtient des gels sclérosants particulièrement stables avec l'éthanol pur ou en solution avec de l'eau tel que décrit ci-dessous.

Ledit solvant est choisi parmi l'éthanol et les mélanges eau-éthanol contenant au moins 50% en volume d'éthanol et de préférence au moins 90% en volume d'éthanol, les mélanges eau éthanol contenant au moins 50% en volume d'éthanol et de préférence au moins 90% en volume d'éthanol. On obtient ainsi avec l'éthylcellulose des gels stables ayant l'action sclérosante de l'éthanol.

Selon un mode de réalisation particulier, le produit comprend, en outre, un deuxième polysaccharide choisi parmi les mucilages dont notamment, l'agar-agar et les pectines et de préférence parmi les pectines et en outre, au moins un radionucléide utilisable en thérapie, de préférence en une quantité telle que la totalité du radionucléide est piégé dans ledit deuxième polysaccharide, le mélange de ladite composition de base, de ladite substance active, dudit deuxième polysaccharide et dudit radionucléide présente une viscosité qui permet l'injection dudit mélange et ledit mélange forme un précipité au contact d'une solution aqueuse éventuellement saline.

Lorsque le produit comporte un radionucléide ou un agent de contraste, ils sont de préférence sous forme divisées (poudre ou granulés) afin de ne pas modifier la viscosité du mélange permettant la formation de l'implant. Le radionucléide peut également être présent en l'absence du deuxième polysaccharide. En effet, la Demanderesse a mis en évidence que le précipité formé compose un réseau tridimensionnel semblable à celui du coton hydrophile. Un radionucléide ou toute autre substance active sous forme de poudre est immobilisé dans le réseau formé par le précipité, ce qui empêche sa migration dans l'organisme du sujet et concentre son activité autour de la zone proche de l'implant. La présence du deuxième polysaccharide permet néanmoins de conforter le maintien du radionucléide dans l'implant. Avantageusement, le produit comporte en outre un agent de contraste et/ou un colorant.

De préférence on utilise une composition de base éthylcellulose éthanol tel que décrit ci-dessus et de la pectine en tant que deuxième polysaccharide. La pectine est capable d'emprisonner des radionucléides, sa présence vient donc renforcer l'action mécanique de piège du réseau formé par le précipité/l'implant.

Le produit peut également comprendre un agent de contraste non radioactif, tel qu'une huile iodée par exemple.

Selon un mode de réalisation, le rapport massique substance active/composition de base est sensiblement égal ou supérieur à 0.15. Dans le cas de l'éosine ou du bleu de méthylène dans une composition de base éthylcellulose-éthanol, on obtient une diffusion satisfaisante de l'éosine ou du bleu de méthylène dans le milieu environnant l'implant, ce dernier demeurant coloré et donc facilement localisable.

Le premier polysaccharide est l'éthylcellulose et ledit solvant est choisi parmi l'éthanol et les mélange eau-éthanol comportant au moins 50% en volume d'éthanol et notamment au moins 90% en volume d'éthanol.

Selon un mode de réalisation qui peut être combiné à l'un quelconque des modes de réalisation précités, le produit comprend, en outre, un dispositif d'injection, ladite quantité de substance active et/ou ledit deuxième polysaccharide éventuellement en mélange avec ledit radionucléide est/sont conditionné(e)s séparément de ladite composition de base, de préférence sous forme de poudre et sont aptes à être mélangés à cette dernière dans ledit dispositif d'injection. Le radionucléide sert d'élément de contraste et permet de traiter, par exemple, les cancers du fait des rayons qu'il émet.

Par dispositif d'injection, on entend, au sens de la présente invention, par exemple, une seringue sur laquelle est montée une aiguille de longueur et de diamètre adaptés au lieu de l'injection, un cathéter sur lequel est montée une aiguille ou un dispositif de brachythérapie.

Le ratio massique deuxième polysaccharide/premier polysaccharide n'est pas limité selon l'invention ; il est par exemple inférieur ou égal à 1/20.

Selon un autre mode de réalisation qui peut être combiné à l'un quelconque des modes de réalisation précités, le produit comprend, de plus, un gaz chimiquement inertes vis-à-vis de ladite composition de base et de ladite substance active, ledit gaz forme un mélange diphasique avec ladite composition de base, ladite substance active, l'éventuel deuxième polysaccharide et l'éventuel radionucléide, le mélange dudit gaz, de ladite composition de base, de ladite substance active, dudit éventuel deuxième polysaccharide et dudit éventuel radionucléide ou agent de contraste ou colorant présente une viscosité qui permet l'injection dudit mélange et ledit mélange forme un précipité au contact d'une solution aqueuse éventuellement saline.

Le gaz forme des bulles qui sont détectables par échographie, par exemple.

Lorsque ladite composition de base comporte de l'hydroxyapatite la quantité de cette dernière n'est pas limitée. Avantageusement, le produit contient une quantité d'hydroxyapatite sensiblement égale ou inférieure la quantité dudit premier polysaccharide. L'hydroxyapatite forme une suspension avec la composition de base et les éventuels autres composants du produit. Lorsque le produit précipite pour former l'implant, l'hydroxyapatite est répartie dans tout le précipité, lequel forme un réseau tridimensionnel apte à être colonisé par des ostéoblastes notamment. Ce type d'implant est adapté la reconstruction osseuse.

La présente invention concerne également une composition pharmaceuticalement acceptable comprenant une composition de base pharmaceuticalement acceptable qui contient au moins un solvant, au moins un premier polysaccharide soluble dans ledit solvant, ladite composition de base est apte à former un précipité au contact d'une solution aqueuse éventuellement saline et qui de manière caractéristique contient
- une quantité donnée d'au moins une substance active sous forme de poudre, qui est choisie parmi:
- les agents bactéricides/antiseptiques/fongiques ioniques et solubles dans ledit solvant tels que l'éosine et le bleu de méthylène et l'iodure de potassium;
- les antibiotiques, les facteurs de croissance, l'hydroxyapatite, les hormones, les anti-tumoraux, les agents antimitotiques, les inhibiteurs de la topo isomérase, les agents anti-cancéreux et les mélanges d'au moins deux desdites substances actives et ladite composition pharmaceutique présente une viscosité qui permet son injection et en ce qu'elle forme un précipité au contact d'une solution aqueuse éventuellement saline.

Avantageusement, la composition peut comprendre un deuxième polysaccharide choisi parmi les mucilages, dont notamment, l'agar-agar et les pectines et de préférence parmi les pectines et est injectable (du fait de sa viscosité).

La composition peut également comprendre, en outre, au moins un radionucléide utilisable en thérapie, et/ou éventuellement un agent de contraste et/ou un colorant et est injectable (du fait de sa viscosité).

Avantageusement, toutes les substances actives, notamment les antibiotiques, les anti-tumoraux et les anticancéreux sont anioniques de manière à sortir du précipité formé par l'implant.

La présente invention concerne également un implant obtenu par précipitation totale ou partielle de la composition précitée.

La présente invention concerne également un procédé de fabrication d'une composition pharmaceuticalement acceptable selon lequel,
- on prépare ladite composition de base ;
- on dissout sous agitation à température ambiante dans ladite composition de base, ladite substance active qui se présente sous forme de poudre et/ou ledit deuxième polysaccharide sous forme de poudre éventuellement mélangé avec ledit radionucléide sous forme de poudre.

Dans toute la présente demande, à titre d'exemple, d'anticancéreux, on peut citer les sels de platine tels que par exemple, le cis• diaminedichloroplatine(II) ou cisplatine, le (R,R)-1,2-diaminocyclohexane (éthanedioate-O,O) platine ou oxaliplatine, le cyclobutane-dicarboxyloplatine ou carboplatine, le [2-(aminomethyl)cyclobutyl]methanamine; 2-oxidopropanoate;platinum(4+) ou lobaplatine, qui sont également opaques aux rayons X et peuvent alors faire office également d'agent de contraste.

On peut également citer, notamment à titre d'agents anticancéreux, les radionucléides choisis parmi le Phosphore 32, Thallium, le Technétium, le Strontium 89, l'Indium, l'Yttrium 90, l'Iode 131, le Holmium 166, le Rhénium 186 et 188, le cuivre 67, le Samarium 153, le Lutétium 177, le Dysprosium 165, Gallium et le Césium et les composés dont les molécules contiennent un atome radioactif choisi notamment parmi les radionucléides précités. Ces substances peuvent également faire office d'agent de contraste.

On peut également citer à titre d'exemples non limitatifs de facteur de croissance l'érythropoïétine, le GM-CSF (« Granulocyte Macrophage Colony Stimulating Factor ») et le GCSF (« Granulocyte Colony Stimulating Factor »).

A titre d'exemples non limitatif d'antibiotiques, peut citer les composés de la famille de la pénicilline qui sont solubles dans l'éthanol tel que par exemple la benzylpénicilline (Pénicilline G), la benzathine benzylpénicilline, la phénoxyméthylpenicilline (pénicilline V), l'aminopénicillines (pénicilline A), les pénicillines résistantes aux pénicillinases ou (pénicillines M), les carboxypénicillines, les uréidopénicillines, les amidinopénicillines. On peut également cité à titre d'exemple d'antibiotiques utilisables dans le cadre de la présente invention, les cyclines, les aminosides, les macrolides, les quinolones, la fucidine (contre les staphylocoques), les lincosamides, les antibiotiques phénicolés, les sulfamides, les combinaisons de sulfamides et de triméthoprime, les synergistines et la forsfomycine. Ces substances seront sous forme de poudre pour parvenir à l'obtention d'un mélange stable.

Le produit de l'invention, la composition de l'invention et l'implant de l'invention peuvent ainsi servir au traitement des vaisseaux sanguins (destruction des vaisseaux à faible débit) au traitement des hernies discales, au traitement des cancers comme le cancer de la thyroïde, le cancer de la prostate, dans la reconstruction osseuse, le traitement des kystes bénins, le traitement des tumeurs bégnines ou malignes bien isolées.

À titre d'exemple, lorsque le produit de l'invention comprend un radio nucléotide, ce dernier est présent en une quantité efficace pour avoir une action thérapeutique. Le volume maximal du mélange injecté peut être de 5ml, par exemple. On obtient ainsi un implant qui présente une activité thérapeutique du fait des rayons gamma qu'il émet et qui est facilement localisable.

Les polysaccharides, en particulier la cellulose et ses dérivés ont des propriétés antibactériennes connues qui peuvent renforcer ou compléter l'activité de la substance et active et/ou du solvant.

Par ailleurs, la Demanderesse a constaté que les mucilages, en particulier les pectines sont capables de séquestrer les métaux lourds et les radionucléides. La présence de pectine(s) ou autre mucilage permet de former un implant qui possède une activité anti-cancéreuse de par le rayonnement gamma du ou des radionucléides piégés dans la pectine ou cet autre mucilage ; les radionucléides sont également des agents de contraste. La zone d'action de l'implant peut être facilement limitée par le volume de l'implant qui est directement déterminé par la quantité de composition pharmaceutique selon l'invention injectée dans l'organisme du sujet. La pose de l'implant est effectuée en même temps que sa formation, c'est-à-dire par injection ; elle est moins invasive que des microcapsules contenant un radionucléide, notamment du fait de la formation du précipité floconneux et donc souple comme précédemment expliqué. De plus, on peut obtenir un implant qui comble et assèche le kyste, rendant également ce dernier plus facilement détectable ou détectable par une autre méthode de détection. On peut également obtenir un implant qui se forme en périphérie du kyste ou de la tumeur et forme une excroissance. L'action thérapeutique est alors obtenue en premier lieu au niveau de la zone de contact entre l'implant et la kyste/tumeur. Dans le cadre d'une tumeur, la présence de l'implant va mobiliser le système immunitaire pour favoriser sa destruction.

La Demanderesse a également mis en évidence que les pectines mélangées à des solutions d'éthyl cellulose peuvent être stérilisées sans destruction majeure du réseau formé par la pectine, ce dernier s'entremêlant avec celui formé par l'éthyl cellulose.

### Définitions

Le terme « injectable » désigne une composition présentant une viscosité mesurée avec un rhéomètre rotatif tel qu'indiqué ci-dessous à 25°C sensiblement égale ou inférieure à 600 Pa.s, de préférence sensiblement égale ou inférieure à 300 Pa.s.

Le terme « substance active» désigne une substance pharmaceuticalement acceptable qui présente un effet médical sur un organisme vivant, cet effet pouvant être thérapeutique (traitement curatif d'une pathologie) ou prophylactique, une substance pouvant améliorer simplement un état lié à une pathologie, comme par exemple, la douleur, une substance qui peut restaurer, corriger ou modifier une fonction physiologique en exerçant une action pharmacologique, immune ou métabolique et une substance pouvant permettre un diagnostic. Les termes dérivés tels que « traitement thérapeutique » désignent l'administration, en particulier par injection, d'une substance active.

Le terme « quantité de substance active » ne désigne pas nécessairement une quantité adaptée pour produire un effet médical mais de préférence correspond à cette quantité.

Le terme « solution aqueuse éventuellement saline » désigne notamment le sang, la lymphe, le sérum, le plasma, le milieu intracellulaire et toute solution de type « sérum physiologique ».

Le terme « agent de contraste » désigne toute substance ou mélange de substances susceptible - par une technique d'imagerie médicale connue où les particules énergétiques peuvent générer des images et mettre en évidence certains tissus organes ou partie du corps humain ou animal telle que, par exemple, la radiographie, l'échographie, l'IRM, la scintigraphie, la tomographie par émission de positons (TEP), la tomographie d'émission mono photonique (TEMP). Il peut s'agir par exemple d'une huile iodée ou de métaux tels que le tungstène et le tantale, par exemple.

Les colorants peuvent aussi permettre de localiser l'implant lorsqu'il est formé/posé sous la peau, par exemple. Ils rendent ainsi l'implant détectable à l'œil.

Le terme « mucilage » désigne toute substance secrétée naturellement par un organisme vivant d'origine végétale, animale ou micro bactérienne et qui gonfle au contact de l'eau en prenant une consistance visqueuse à température ambiante (25°C). A titre d'exemple de mucilage, on peut citer les pectines, le mucilage obtenu à partir des graines de lin ou de plantain, les mucilages produits par le goémon blanc, l'agar-agar et l'agarose.

Dans toute la présente demande, la viscosité indiquée est la valeur obtenue par mesure à 25°C au moyen d'un rhéomètre rotatif ARES commercialisé par la société TA instruments et équipé d'une géométrie coaxiales en acier inoxydable. Le diamètre du cylindre intérieur est de 16,5 mm, le diamètre du cylindre extérieur est de 17mm et la hauteur de 13mm. Les mesures sont faites à 25°C, le rhéomètre ayant été porté à cette température avant les mesures. Les valeurs indiquées permettent d'obtenir une composition injectable dans l'organisme d'un sujet, éventuellement après chauffage de la composition pour la rendre plus fluide.

Le terme « pectines » désigne des polysaccharides comprenant un squelette d'acide a-D-galacturonique et de faibles quantités de a-L-rhamnose plus ou moins ramifiés. Il englobe les polysaccharides dont la fonction carboxyle des acides a-D-galacturoniques est sous forme acide, ionisée, par exemple par le calcium, ou forme un ester, par exemple avec le méthanol et les polysaccharides dont les acides galacturoniques sont acétylés. Ainsi, selon l'invention le terme « pectines » regroupe les acides pectiques qui présentent un degré de méthylation inférieur à 5% (DM<5), les pectines faiblement méthylées qui présentent un degré de méthylation inférieur à 50% (DM<50) et les pectines hautement méthylées qui présentent un degré de méthylation supérieur à 50%. Le poids moléculaire n'est pas limité selon l'invention de même que l'origine des pectines. Il peut s'agir, par exemple de pectine de pomme, de citron ou de betterave.

Le terme « sujet » désigne un animal ou un être humain, adulte ou non et plus particulièrement un animal ou un être humain dont l'état de santé nécessite un traitement thérapeutique.

Le terme « pharmaceuticalement acceptable » désigne une substance qui, lorsqu'elle est introduite dans l'organisme du sujet ne cause pas d'effet toxique et/ou d'effet secondaire ayant une intensité comparable ou supérieure à l'effet médical obtenu par utilisation de la substance active. Dans le cas du traitement des cancers, une composition pharmaceuticalement acceptable permet de tuer les cellules cancéreuses sans que les effets secondaires soient supérieurs aux bénéfices thérapeutiques apportés par l'administration de la composition.

Le terme « injection » recouvre les injections sous cutanées, intraveineuses, intramusculaires, intra-osseuses, percutanées (sous-cutanée intra-dermique) et intra (organe ou tissu spécifique).

Le terme « éosine » englobe les deux formes Y et B de l'éosine ainsi que leurs mélanges.

### Figures

La présente invention, ses caractéristiques et les divers avantages qu'elle procure apparaîtront mieux à la lecture de la description qui suit et fait références aux dessins annexés sur lesquels :
- la Fig. 1 représente sur la courbe du haut, la viscosité des gels d'éthanol à 96° et d'éthylcellulose en fonction de la concentration en éthanol , la courbe du bas représente le % massique d'éthanol du gel ;
- la Fig. 2 est une photographie qui représente le dispositif expérimental utilisé pour mesurer le passage de l'éthanol et de l'éosine ou du bleu de méthylène vers l'extérieur du précipité qui va former partiellement l'implant après injection du produit de l'invention ;
- la Fig. 3a est une photographie du précipité obtenu avec de l'éosine et la Fig. 3b de celui obtenu avec du bleu de méthylène ; et
- la Fig. 4 est une photographie du précipité obtenu à partir d'une composition de base qui est une suspension d'une poudre métallique dans la composition de base éthanol à 96°-éthylcellulose.

### EXEMPLES

### Préparation de la composition de base

### Ingrédients :

Ethylcellulose référence Aqualon N100F, grade pharmaceutique, % éthoxy =49.2 ; viscosité intrinsèque à 25°C=94mPa-S dans l'éthanol selon la méthode normée de la pharmacopée Européenne commercialisée par la société ASHLAND/SPCI.

Ethanol à 96° (solution contenant de l'eau et de l'éthanol) Ethanol 96%, qualité pharmaceutique, D= 0.81 Kg/L, commercialisé par la société Fisher Chemicals.

Plusieurs gels d'éthanol à 96° et d'éthylcellulose ont été préparés par dilution avec de l'éthanol à 96° d'une composition mère constituée de 1 600 ml d'éthanol à 96° et de 94,25 g d'éthylcellulose. La viscosité de ces solutions a été mesurée à 25°C à l'aide du rhéomètre précité.

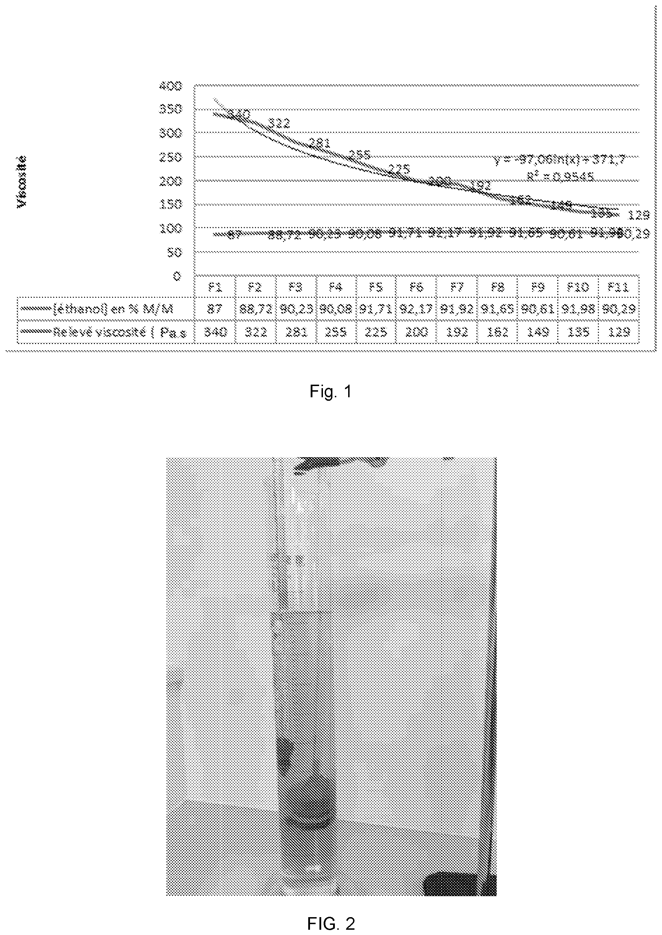

La Fig. 1 représente la viscosité de ces solutions en fonction de la concentration massique d'éthylcellulose (masse d'éthylcellulose / (masse d'éthylcellulose + masse d'éthanol à 96°). Toutes ces solutions présentent une viscosité qui les rend injectables.

### Ajout d'une substance active sous forme de poudre

Dans 10 ml de chacun des gels préparés à l'étape précédente, on a dissout à température ambiante et sous agitation faible, 1.5g d'éosine Y en poudre (commercialisée par la société RAL diagnostic) ou 1.5g de bleu de méthylène (commercialisé par la société Reag Ph. Eur) en poudre. On a vérifié que la viscosité de ces compositions reste dans la même gamme que celle des gels de base.

On a préparé du sérum physiologique (eau salée à 9/1 000 soit 9g/l) -- la concentration du sérum sanguin étant de 6g/l, les 3g supplémentaires visent à compenser l'osmolarité des autres ions du sang présents. On a placé dans un osmoseur monté sur potence et équipé d'une membrane hémiperméable, le gel contenant le bleu de méthylène ou l'éosine précité. La membrane hémiperméable a été placée dans une éprouvette graduée contenant 400ml du sérum physiologique précité. À l'aide d'un spectrophotomètre réglé sur la longueur d'onde correspondant au pic d'absorbance le plus élevé du bleu de méthylène calé à =668nm ou sur la longueur d'onde correspondant au pic d'absorbance le plus élevé de l'éosine Y à À=525nm on a déterminé le passage de l'éosine ou du bleu de méthylène du gel vers le sérum physiologique. De même, à l'aide d'un alcoomètre 0 à 100% équipé d'un thermomètre et commercialisé par la société « A|Ambik^{®}, on a mesuré le passage de l'éthanol du gel vers le sérum à travers la membrane hémiperméable.

La membrane hémiperméable est une modélisation comportementale de la membrane plasmique cellulaire. Les cellules constituent les parois vasculaires, kystiques, les tumeurs, ou tout autre tissu à traiter par l'implant selon l'invention. Lorsqu'une substance passe à travers la membrane hémiperméable cela signifie que d'une part cette substance sort du précipité formé par le produit et que d'autre part, elle peut passer par osmose à travers la membrane cellulaire.

On a d'abord observé la formation d'un précipité au niveau de la membrane hémiperméable. Le gel contenant de l'éosine ou du bleu de méthylène forme bien un précipité comme la composition de base au contact du sérum physiologique précité. Au-dessus du précipité, la composition reste sous forme de gel. L'implant formé *in situ* est donc souple et déformable. Il ne va pas causer de dommages mécaniques aux tissus environnants. Par ailleurs, l'osmomètre indique une pression osmotique ce qui confirme la diffusion de certains composés à travers la membrane hémiperméable.

On a ensuite observé la diffusion de l'éosine vers le sérum physiologique. Comme cela est visible sur la Fig. 2, l'éosine traverse la membrane hémiperméable et passe dans le sérum physiologique comme en atteste la couleur rouge de ce dernier. La diffusion de l'éosine ou du bleu de méthylène débute 30 minutes après le contact de la membrane hémiperméable avec le sérum physiologique. 12h après ce contact, la pression osmotique est quasi nulle ce qui indique la fin du passage de l'éosine ou du bleu de méthylène dans le sérum physiologique.

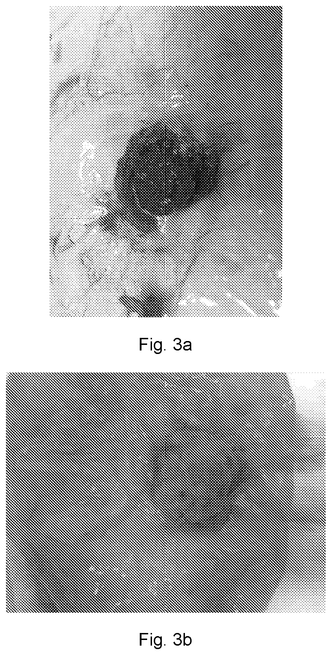

La Fig. 3 montre bien que le précipité obtenu contient de l'éosine qui lui confère sa couleur rouge.

On a ensuite déterminé le passage de l'éthanol du gel à travers la membrane hémiperméable, à l'aide de l'alcoomètre disposé dans l'éprouvette graduée. Au bout de 30 minutes à compter de l'immersion de l'osmomètre dans le sérum physiologique, on n'observe aucune variation de la concentration d'éthanol dans l'éprouvette graduée. Il en est de même au bout de 12h. Le même phénomène est observé avec du bleu de méthylène. Par ailleurs, le précipité obtenu reste coloré même après passage de l'éosine ou du bleu de méthylène dans le sérum physiologique.

La Demanderesse a ainsi mis en évidence que l'éthanol demeurait dans le précipité, lui conférant une action sclérosante durable et que le bleu de méthylène ou l'éosine diffusait hors du précipité permettant ainsi de désinfecter l'environnement de l'implant.

### Exemple 1:. préparation d'une composition pharmaceuticalement acceptable selon l'invention pour son utilisation dans le traitement de l'ostéomyélite

Préparation de la composition de base : on mélange 100 ml d'éthanol à 96° avec 10 grammes d'éthylcellulose à 20°C.

On ajoute ensuite à la composition de base précitée 0,1 d de poudre de bleu de méthylène ou d'éosine et 5 grammes d'hydroxyapatite en poudre (hydroxyapatite sous forme de poudre pureté >95% commercialisée par la société MIDICOAT; poudre BTE/5-183). On obtient une composition présentant une viscosité identique à celle de la composition de base contenant l'éthylcellulose et l'éthanol à 96°.

Le mélange obtenu peut être injecté dans un kyste osseux.

L'éthanol bactéricide contribue à la désinfection du lieu de l'injection. On observe une synergie d'action bactéricide avec le colorant.

Le précipité d'éthylcellulose forme alors une matrice dans le kyste osseux qui va permettre la colonisation des ostéoblastes nécessaires à la formation d'os

### Exemple 2: préparation d'une composition pharmaceuticalement acceptable selon l'invention pour son utilisation dans la reconstruction osseuse

On prépare une composition de base comme expliqué dans l'Exemple 1 précité. On ajoute à cette composition de base un facteur de croissance tel que précité et 5 g d'hydroxyapatite.

Le mélange obtenu peut être dans une cavité formée dans un tissu osseux. Le facteur de croissance et l'hydroxyapatite permettent la colonisation de l'implant spongieux par les ostéoblastes ce qui contribue à la formation de matière osseuse.

### Exemple 3: préparation d'une composition pharmaceuticalement acceptable selon l'invention pour son utilisation dans le traitement des kystes bénins ou tumeurs bégnines

On prépare une composition de base comme indiqué en référence à l'Exemple 1. On ajoute ensuite un colorant (bleu de méthylène). L'éthanol présente ici un effet thérapeutique car il va assécher le kyste pour engager un début de nécrose. Le colorant permet de localiser les contours du kyste, le précipité s'étant formé autour de ce dernier et permettre l'exérèse du kyste. L'éthylcellulose permet de former un implant solide localisé qui emprisonne le solvant et le colorant et limite donc leur diffusion dans l'organisme.

### Exemple 4 : préparation d'une composition pharmaceuticalement acceptable selon l'invention contenant de la pectine

On prépare une composition de base en mélangeant 4,2500 g d'éthylcellulose avec 67,4105 g d'éthanol à 96°. Les ingrédients sont identiques à ceux précités en référence à la Fig. 1. On ajoute à ce mélange 5g de pectine grade pharmaceutique commercialisée par la société Coopération Pharmaceutique Française. On observe que sous agitation à 18C on obtient un mélange homogène. Ce mélange est un gel dont la viscosité mesurée comme en référence à la Fig. 1 est sensiblement proche de celle du gel d'éthanol à 96° et d'éthylcellulose. Ce gel reste donc injectable.

Au contact d'une solution saline telle que du sérum physiologique tel que précédemment décrit, on obtient un précipité cotonneux, déformable et légèrement élastique qui n'est pas dégradé lorsqu'il est chauffé à une température de 40°C.

Après 5 jours, le mélange obtenu devient hétérogène et l'on observe que la pectine se dépose au fond du récipient contenant le mélange.

Des essais de dissolution de pectine dans l'éthanol à 96° ont montré que cette dernière n'était pas soluble dans l'éthanol à 96°.

### Détermination de la quantité de pectine ajoutée maximale permettant d'obtenir un gel stable

On a élaboré une gamme de gels (A, B et C) obtenus par mélange d'éthanol à 96° avec de l'éthylcellulose chacun des gels présentant une viscosité et une densité différente.

Le Tableau Il regroupe les différentes compositions des gels.

**Tableau Il**

| | Gel b | GeIC | Gel D | Gel E |
|---|---|---|---|---|
| Ethylcellulose (g) | 7,27 | 10 | 20 | 10 |
| Ethanol 96°(ml) | 100 | 100 | 100 | 100 |
| Pectine (g) | 5 | 0,5 | 1 | 0,1 |

Les trois gels présentent une viscosité permettant de les injecter dans le corps d'un sujet.

La pectine apparaît bien en suspension stable dans le gel D et dans le gel E. Il n'en est rien pour les gels B et C pour lesquels la pectine se sépare du gel au bout de 24h.

Il semble donc que pour des gels éthanol à 96°-éthylcellulose présentant une viscosité supérieure à 340mPa-S (et de densité optimale du gel) que la pectine soit plus facilement en suspension stable sans toutefois dépasser le rapport

### Masse (Pectine)/Masse (éthylcellulose) expérimental de 1/20.

Les particules de pectine pulvérisée utilisées dans cette série d'expérience peuvent être encore plus fines, ce qui peut encore accroître la stabilité de leur suspension dans le mélange. Une agitation vigoureuse (agitation à l'aide d'un agitateur excentré de type Vortex) permet une remise ne suspension du mélange d'éthanol gélifié + pectine. L'Homme du Métier est à même de choisir la granulométrie la plus appropriée.

L'incorporation de poudre d'éosine Y ou de bleu de méthylène dans le mélange éthanol gélifié+ pectine s'effectue à froid sans aucune difficulté.

Le mélange coloré est obtenu rapidement et est stable.

Le chauffage des différents mélanges obtenus montrent qu'ils résistent à la chaleur. Le gel semble avoir un comportement élastique ; c'est-à-dire que la viscosité avant chauffage est obtenue de nouveau une fois le gel refroidi à température initiale.

La mise au contact de ce gel coloré (éthylcellulose/éthanol à 96°/pectine) dans un milieu aqueux provoque une précipitation immédiate. La diffusion de l'éthanol et de l'éosine ou du bleu de méthylène est la même que celle observé pour l'expérience en référence à la Fig. 2.

### Exemple 5 préparation selon l'invention permettant le traitement des tumeurs cancéreuses

On prépare une composition de base contenant de l'éthylcellulose, de l'éthanol à 96° et de la pectine comme indiqué en référence à l'exemple 4 avec un rapport masse de pectine/masse d'éthylcellulose égal à 1/20. On ajoute ensuite un colorant (bleu de méthylène), et un radionucléide en poudre.

L'éthanol présente également un effet thérapeutique car il va nécroser la tumeur. Le radionucléide sert à la fois de substance active du fait des rayons gamma qu'il émet et permet également la localisation de l'implant formé par précipitation. La pectine va renforcer l'action de l'éthylcellulose en emprisonnant le radionucléide.

### Exemple 6 : préparation selon l'invention permettant le traitement des tumeurs cancéreuses sans pectine

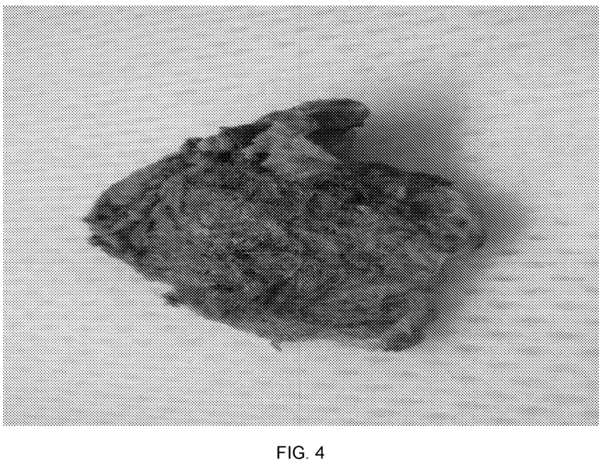

On a préparé une composition de base en mélangeant 130mg d'éthyl cellulose avec 2095,6 mg d'éthanol à 96°. On a ajouté dans cette composition de base 300mg d'une poudre métallique simulant un radionucléide. Comme cela est visible sur la Fig. 4, le précipité est une masse tridimensionnelle formant un réseau de fibres tel que du coton et qui emprisonne dans tout son volume la poudre métallique. Cet exemple montre que le radionucléide peut être utilisé sans la pectine.

## Revendications

1. Produit permettant par injection la formation d'un implant solide dans le corps d'un sujet, ledit produit comprenant une composition de base, pharmaceuticalement acceptable, qui contient au moins un solvant et au moins un premier polysaccharide soluble dans ledit solvant, ladite composition de base est apte à former un précipité au contact d'une solution aqueuse éventuellement saline ;
**caractérisé en ce qu'**il comprend, en outre, une quantité donnée d'au moins une substance active, pharmaceuticalement acceptable sous forme de poudre, choisie parmi :
- les agents bactéricides/antiseptiques/antifongiques ioniques et solubles dans ledit solvant tels que l'éosine et le bleu de méthylène et l'iodure de potassium ;
- les antibiotiques, les facteurs de croissance, l'hydroxyapatite, les hormones, les agents anti tumoraux, les agents antimitotiques, les inhibiteurs de la topo isomérase, les agents anti-cancéreux et les mélanges d'au moins deux desdites substances actives; et
**en ce que** ladite quantité de substance active est mélangée à ladite composition de base ou conditionnée séparément de ladite composition de base,
**en ce que** le mélange formé par ladite composition de base et ladite quantité de substance active présente une viscosité qui permet l'injection dudit mélange et **en ce que** ledit mélange forme un précipité au contact d'une solution aqueuse éventuellement saline permettant de former ledit implant dans l'organisme du sujet dans lequel il est injecté, et de diffuser ladite substance active dans un milieu environnant l'implant ; et
**en ce que** ledit premier polysaccharide est l'éthylcellulose et ledit solvant est choisi parmi l'éthanol et les mélange eau-éthanol comportant au moins 50% en volume d'éthanol et notamment au moins 90% en volume d'éthanol.

2. Produit selon la revendication 1, **caractérisé en ce que** ladite substance active est sous ladite forme de poudre et est choisi parmi :
- les agents bactéricides/antiseptiques/antifongiques ioniques et solubles dans ledit solvant tels que l'éosine et le bleu de méthylène et l'iodure de potassium ;
- les antibiotiques, l'hydroxyapatite, et les mélanges d'au moins deux desdites substances actives.

3. Produit selon la revendication 1, **caractérisé en ce que** ladite substance active est sous ladite forme de poudre et est choisi parmi les agents bactéricides/antiseptiques/antifongiques ioniques et solubles dans ledit solvant tels que l'éosine et le bleu de méthylène et l'iodure de potassium et **en ce qu'**il comprend, en outre :
- un deuxième polysaccharide choisi parmi les pectines, et pour lequel le ratio massique deuxième polysaccharide/premier polysaccharide est inférieur ou égal à 1 /20, et
- au moins un radionucléide utilisable en thérapie, en une quantité telle que la totalité du radionucléide est piégée dans ledit deuxième polysaccharide, et **en ce que** le mélange de ladite composition de base, de ladite substance active, dudit deuxième polysaccharide et dudit radionucléide présente une viscosité qui permet l'injection dudit mélange et **en ce que** ledit mélange forme un précipité au contact d'une solution aqueuse éventuellement saline.

4. Produit selon l'une quelconque des revendications précédentes, **caractérisé en ce qu'**il comporte en outre un agent de contraste et/ou un colorant.

5. Produit selon l'une quelconque des revendications précédentes, **caractérisé en ce que** le rapport massique substance active/composition de base est sensiblement égal ou supérieur à 0.15.

6. Produit selon l'une quelconque des revendications précédentes, **caractérisé en ce qu'**il comprend, en outre, un dispositif d'injection, **en ce que** ladite quantité de substance active et/ou ledit deuxième polysaccharide éventuellement en mélange avec ledit radionucléide est/sont conditionnées séparément de ladite composition de base, de préférence sous forme de poudre et sont aptes à être mélangés à cette dernière dans ledit dispositif d'injection.

7. Produit selon l'une quelconque des revendications précédentes, **caractérisé en ce qu'**il comprend, de plus, un gaz chimiquement inertes vis-à-vis de ladite composition de base et de ladite substance active, **en ce que** ledit gaz forme un mélange diphasique avec ladite composition de base, ladite substance active, l'éventuel deuxième polysaccharide et l'éventuel radionucléide, **en ce que** le mélange dudit gaz, de ladite composition de base, de ladite substance active, dudit éventuel deuxième polysaccharide et dudit éventuel radionucléide présente une viscosité qui permet l'injection dudit mélange et **en ce que** ledit mélange forme un précipité au contact d'une solution aqueuse éventuellement saline.

8. Produit selon l'une quelconque des revendications précédentes, **caractérisé en ce que** ladite composition de base comporte de l'hydroxyapatite et notamment en une quantité sensiblement égale ou inférieure la quantité dudit premier polysaccharide.

9. Composition pharmaceuticalement acceptable comprenant une composition de base pharmaceuticalement acceptable qui contient au moins un solvant, au moins un premier polysaccharide soluble dans ledit solvant, ladite composition de base est apte à former un précipité au contact d'une solution aqueuse éventuellement saline ; **caractérisée en ce qu'**elle comprend
- une quantité donnée d'au moins une substance active sous forme de poudre, qui est choisie parmi :
- les agents bactéricides/antiseptiques/fongiques ioniques et solubles dans ledit solvant tels que notamment l'éosine et le bleu de méthylène et l'iodure de potassium ;
- les antibiotiques, les facteurs de croissance, l'hydroxyapatite, les hormones,
- les anti tumoraux, les agents antimitotiques, les inhibiteurs de la topo isomérase, les agents anti-cancéreux et les mélanges d'au moins deux desdites substances actives et **en ce que** ladite composition pharmaceutique présente une viscosité qui permet son injection et **en ce qu'**elle forme un précipité au contact d'une solution aqueuse éventuellement saline permettant de former un implant dans l'organisme du sujet dans lequel il est injecté et permettant de diffuser ladite substance active dans un milieu environnant l'implant, et **en ce que**
ledit premier polysaccharide est l'éthylcellulose et ledit solvant est choisi parmi l'éthanol et les mélange eau-éthanol comportant au moins 50% en volume d'éthanol et notamment au moins 90% en volume d'éthanol.

10. Composition selon la revendication 9, caractérisée en ce ladite substance active qui est sous forme de poudre, contient en outre,
un deuxième polysaccharide choisi parmi les mucilages, dont notamment, l'agar-agar et les pectines et de préférence parmi les pectines et en ce qu'elle est injectable et/ou en ce qu'elle contient, en outre au moins un radionucléide utilisable en thérapie, et/ou un agent de contraste et/ou un colorant et en ce qu'elle est injectable.

11. Implant obtenu par précipitation totale ou partielle de la composition selon la revendication 9 ou 10.

12. Procédé de fabrication d'une composition pharmaceuticalement acceptable selon la revendication 9 à 10, **caractérisé en ce que** :
- l'on prépare ladite composition de base ;
- on dissout sous agitation à température ambiante dans ladite composition de base, ladite substance active ou lesdites substances actives qui se présente(nt) sous forme de poudre et/ou ledit deuxième polysaccharide sous forme de poudre éventuellement mélangé avec ledit radionucléide également sous forme de poudre.

## Patentansprüche

1. Produkt, das durch Injektion das Bilden eines festen Implantats im Körper eines Patienten ermöglicht, wobei das Produkt eine pharmazeutisch verträgliche Basiszusammensetzung umfasst, die mindestens ein Lösungsmittel und mindestens ein erstes in dem Lösungsmittel lösliches Polysaccharid enthält, wobei die Basiszusammensetzung dazu geeignet ist, bei Kontakt mit einer wässrigen Lösung, gegebenenfalls einer Kochsalzlösung, eine Ausfällung zu bilden;
**dadurch gekennzeichnet, dass** es ferner eine gegebene Menge mindestens eines pharmazeutisch verträglichen Wirkstoffs in Pulverform enthält, der aus Folgendem ausgewählt ist:
- bakteriziden/antiseptischen/antimykotischen Mitteln, die ionisch und in dem Lösungsmittel löslich sind, wie Eosin und Methylenblau und Kaliumiodid;
- Antibiotika, Wachstumsfaktoren, Hydroxylapatit, Hormonen, Antitumormitteln, Antimitotika, Topo-Isomerase-Inhibitoren, Antikrebsmitteln und Mischungen aus mindestens zwei der Wirkstoffe; und
dass die Wirkstoffmenge mit der Basiszusammensetzung vermischt oder getrennt von der Basiszusammensetzung verpackt wird,
dass die aus der Basiszusammensetzung und der Wirkstoffmenge gebildete Mischung eine Viskosität aufweist, die die Injektion der Mischung ermöglicht, und dass die Mischung bei Kontakt mit einer wässrigen Lösung, gegebenenfalls einer Kochsalzlösung, eine Ausfällung bildet, die es ermöglicht, das Implantat im Organismus des Patienten, in dem sie injiziert wird, zu bilden und den Wirkstoff in einer das Implantat umgebenden Umgebung zu verteilen; und
dass das erste Polysaccharid Ethylcellulose ist und das Lösungsmittel aus Ethanol und Wasser-Ethanol-Mischungen ausgewählt wird, die mindestens 50 Vol.-% Ethanol und insbesondere mindestens 90 Vol.-% Ethanol umfassen.

2. Produkt nach Anspruch 1, **dadurch gekennzeichnet, dass** der Wirkstoff in Pulverform vorliegt und aus Folgendem ausgewählt wird:
- bakteriziden/antiseptischen/antimykotischen Mitteln, die ionisch und in dem Lösungsmittel löslich sind, wie Eosin und Methylenblau und Kaliumiodid;
- Antibiotika, Hydroxylapatit und Mischungen aus mindestens zwei der Wirkstoffe.

3. Produkt nach Anspruch 1, **dadurch gekennzeichnet, dass** der Wirkstoff in Pulverform vorliegt und aus bakteriziden/antiseptischen/antimykotischen Mitteln, die ionisch und in dem Lösungsmittel löslich sind, wie Eosin und Methylenblau und Kaliumiodid ausgewählt wird,
und dass es ferner Folgendes umfasst:
- ein zweites Polysaccharid, das aus Pektinen ausgewählt wird, und bei dem das Massenverhältnis des zweiten Polysaccharids zum ersten Polysaccharid kleiner gleich 1/20 ist, und
- mindestens ein in der Therapie verwendbares Radionuklid in einer derartigen Menge, dass die Gesamtmenge des Radionuklids im zweiten Polysaccharid eingeschlossen wird, und dass die Mischung aus der Basiszusammensetzung, dem Wirkstoff, dem zweiten Polysaccharid und dem Radionuklid eine Viskosität aufweist, die die Injektion der Mischung ermöglicht, und dass die Mischung bei Kontakt mit einer wässrigen Lösung, gegebenenfalls einer Kochsalzlösung, eine Ausfällung bildet.

4. Produkt nach einem der vorhergehenden Ansprüche, **dadurch gekennzeichnet, dass** es ferner ein Kontrastmittel und/oder einen Farbstoff umfasst.

5. Produkt nach einem der vorhergehenden Ansprüche, **dadurch gekennzeichnet, dass** das Massenverhältnis des Wirkstoffs zur Basiszusammensetzung im Wesentlichen größer gleich 0,15 ist.

6. Produkt nach einem der vorhergehenden Ansprüche, **dadurch gekennzeichnet, dass** es ferner eine Injektionsvorrichtung umfasst, dass die Menge an Wirkstoff und/oder das zweite Polysaccharid, gegebenenfalls mit dem Radionuklid vermischt, getrennt von der Basiszusammensetzung, vorzugsweise in Pulverform, verpackt ist/sind und dazu geeignet sind, mit letzterer in der Injektionsvorrichtung vermischt zu werden.

7. Produkt nach einem der vorhergehenden Ansprüche, **dadurch gekennzeichnet, dass** es außerdem ein Gas umfasst, das gegenüber der Basiszusammensetzung und dem Wirkstoff chemisch inert ist, dass das Gas mit der Basiszusammensetzung, dem Wirkstoff, dem möglichen zweiten Polysaccharid und dem möglichen Radionuklid ein zweiphasige Mischung bildet, dass die Mischung aus dem Gas, der Basiszusammensetzung, dem Wirkstoff, dem möglichen zweiten Polysaccharid und dem möglichen Radionuklid eine Viskosität aufweist, die die Injektion der Mischung ermöglicht, und dass die Mischung bei Kontakt mit einer wässrigen Lösung, gegebenenfalls einer Kochsalzlösung, eine Ausfällung bildet.

8. Produkt nach einem der vorhergehenden Ansprüche, **dadurch gekennzeichnet, dass** die Basiszusammensetzung Hydroxylapatit umfasst, insbesondere in einer Menge, die im Wesentlichen kleiner gleich der Menge des ersten Polysaccharids ist.

9. Pharmazeutisch verträgliche Zusammensetzung, die eine pharmazeutisch verträgliche Basiszusammensetzung umfasst, die mindestens ein Lösungsmittel und mindestens ein erstes in dem Lösungsmittel lösliches Polysaccharid enthält, wobei die Basiszusammensetzung dazu geeignet ist, bei Kontakt mit einer wässrigen Lösung, gegebenenfalls einer Kochsalzlösung, eine Ausfällung zu bilden; **dadurch gekennzeichnet, dass** sie Folgendes umfasst:
- eine gegebene Menge mindestens eines Wirkstoffs in Pulverform, der aus Folgendem ausgewählt ist:
- bakteriziden/antiseptischen/antimykotischen Mitteln, die ionisch und in dem Lösungsmittel löslich sind, wie Eosin und Methylenblau und Kaliumiodid;
- Antibiotika, Wachstumsfaktoren, Hydroxylapatit, Hormonen,
- Antitumormitteln, Antimitotika, Topo-Isomerase-Inhibitoren, Antikrebsmitteln und Mischungen aus mindestens zwei der Wirkstoffe, und dass die pharmazeutische Zusammensetzung eine Viskosität aufweist, die ihre Injektion ermöglicht, und dass sie bei Kontakt mit einer wässrigen Lösung, gegebenenfalls einer Kochsalzlösung, eine Ausfällung bildet, die es ermöglicht, ein Implantat im Organismus des Patienten, in dem sie injiziert wird, zu bilden, und es ermöglicht, den Wirkstoff in einer das Implantat umgebenden Umgebung zu verteilen, und dass
das erste Polysaccharid Ethylcellulose ist und das Lösungsmittel aus Ethanol und Wasser-Ethanol-Mischungen ausgewählt wird, die mindestens 50 Vol.-% Ethanol und insbesondere mindestens 90 Vol.-% Ethanol umfassen.

10. Zusammensetzung nach Anspruch 9, **dadurch gekennzeichnet, dass** der Wirkstoff in Pulverform vorliegt und ferner ein zweites Polysaccharid enthält, das aus Schleimstoffen, darunter insbesondere Agar-Agar und Pektinen, und vorzugsweise aus Pektinen ausgewählt ist, und dass sie injizierbar ist und/oder dass sie ferner mindestens ein in der Therapie verwendbares Radionuklid und/oder ein Kontrastmittel und/oder einen Farbstoff enthält und dass sie injizierbar ist.

11. Implantat, das durch vollständiges oder teilweises Ausfällen der Zusammensetzung nach Anspruch 9 oder 10 erhalten wird.

12. Verfahren zum Herstellen einer pharmazeutisch verträglichen Zusammensetzung nach den Ansprüchen 9 bis 10, **dadurch gekennzeichnet, dass**:
- die Basiszusammensetzung vorbereitet wird;
- der Wirkstoff oder die Wirkstoffe, der bzw. die in Pulverform vorliegen, und/oder das zweite Polysaccharid in Pulverform, gegebenenfalls mit dem Radionuklid vermischt, ebenfalls in Pulverform, unter Rühren bei Raumtemperatur in der Basiszusammensetzung gelöst werden.

## Claims

1. A product allowing by injection the formation of a solid implant in the body of a subject, said product comprising a pharmaceutically acceptable base composition which contains at least one solvent and at least one first polysaccharide soluble in said solvent, said base composition is capable of forming a precipitate upon contact with an aqueous solution, which is possibly saline; **characterised in that** it further comprises a given amount of at least one active substance, which is pharmaceutically acceptable in powder form, selected from:
- ionic bactericidal/antiseptic/antifungal agents soluble in said solvent such as eosin and methylene blue and potassium iodide;
- antibiotics, growth factors, hydroxyapatite, hormones, antitumour agents, antimitotic agents, topoisomerase inhibitors, anticancer agents and mixtures of two or more of said active substances; and
**in that** said amount of active substance is mixed with said base composition or packaged separately from said base composition, **in that** the mixture formed by said base composition and said amount of active substance has a viscosity which allows the injection of said mixture and **in that** said mixture forms a precipitate upon contact with an aqueous solution, which is possibly saline, allowing to form said implant in the body of the subject into which it is injected, and to diffuse said active substance into a medium surrounding the implant; and
**in that** said first polysaccharide is ethylcellulose and said solvent is selected from ethanol and water-ethanol mixtures including at least 50% by volume of ethanol and in particular at least 90% by volume of ethanol.

2. The product according to claim 1, **characterised in that** that said active substance is in said powder form and is selected from:
- ionic bactericidal/antiseptic/antifungal agents soluble in said solvent such as eosin and methylene blue and potassium iodide;
- antibiotics, hydroxyapatite, and mixtures of at least two of said active substances.

3. The product according to claim 1, **characterised in that** said active substance is in said powder form and is selected from ionic bactericidal/antiseptic/antifungal agents soluble in said solvent such as eosin and methylene blue and potassium iodide and **in that** it further comprises:
- a second polysaccharide selected from pectins, and for which the second polysaccharide/first polysaccharide mass ratio is less than or equal to 1/20, and
- at least one radionuclide usable in therapy, in an amount such that all of the radionuclide is trapped in said second polysaccharide, and **in that** the mixture of said base composition, said active substance, said second polysaccharide and said radionuclide has a viscosity which allows the injection of said mixture and **in that** said mixture forms a precipitate upon contact with an aqueous solution, which is possibly saline.

4. The product according to any one of the preceding claims, **characterised in that** it further includes a contrast agent and/or a dye.

5. The product according to any one of the preceding claims, **characterised in that** the mass ratio of active substance/base composition is substantially equal to or greater than 0.15.

6. The product according to any one of the preceding claims, **characterised in that** it further comprises an injection device, **in that** said amount of active substance and/or said second polysaccharide optionally mixed with said radionuclide is/are packaged separately from said base composition, preferably in powder form and are capable of being mixed with the latter in said injection device.

7. The product according to any one of the preceding claims, **characterised in that** it further comprises a gas chemically inert with respect to said base composition and said active substance, **in that** said gas forms a two-phase mixture with said base composition, said active substance, the possible second polysaccharide and the possible radionuclide, **in that** the mixture of said gas, said base composition, said active substance, said possible second polysaccharide and said possible radionuclide has a viscosity which allows the injection of said mixture and **in that** said mixture forms a precipitate upon contact with an aqueous solution which is possibly saline.

8. The product according to any one of the preceding claims, **characterised in that** said base composition includes hydroxyapatite and in particular in an amount substantially equal to or less than the amount of said first polysaccharide.

9. A pharmaceutically acceptable composition comprising a pharmaceutically acceptable base composition which contains at least one solvent, at least one first polysaccharide soluble in said solvent, said base composition is capable of forming a precipitate upon contact with an aqueous solution, which is optionally saline; **characterised in that** it comprises
- a given amount of at least one active substance in powder form, which is selected from:
- ionic bactericidal/antiseptic/fungal agents soluble in said solvent such as in particular eosin and methylene blue and potassium iodide;
- antibiotics, growth factors, hydroxyapatite, hormones,
- antitumour agents, antimitotic agents, topoisomerase inhibitors, anticancer agents and mixtures of at least two of said active substances and **in that** said pharmaceutical composition has a viscosity which allows its injection and **in that** it forms a precipitate upon contact with an aqueous solution, which is possibly saline, allowing to form an implant in the body of the subject into which it is injected and allowing to diffuse said active substance in a medium surrounding the implant, and **in that** said first polysaccharide is ethylcellulose and said solvent is selected from ethanol and water-ethanol mixtures including at least 50% by volume of ethanol and in particular at least 90% by volume of ethanol.

10. The composition according to claim 9, **characterised in that** said active substance which is in powder form, further contains a second polysaccharide selected from mucilages, including in particular agar-agar and pectins and preferably from pectins and **in that** it is injectable and/or in that it further contains at least one radionuclide usable in therapy, and/or a contrast agent and/or a dye and **in that** it is injectable.

11. An implant obtained by total or partial precipitation of the composition according to claim 9 or 10.

12. A method for manufacturing a pharmaceutically acceptable composition according to claim 9 to 10, **characterised in that**:
- said base composition is prepared;
- said active substance or active substances which are in powder form and/or said second polysaccharide in powder form, optionally mixed with said radionuclide also in powder form, are dissolved with stirring at room temperature in said base composition.
